# FASCICULE DE BREVET EUROPEEN

(11) **EP 1 465 642 B1**
(45) Date de publication et mention de la délivrance du brevet: **29.02.2012**
(21) Numéro de dépôt: 03712239.7
(22) Date de dépôt: 14.01.2003
(51) Int. Cl.: A61K 33/06, A61K 33/10, A61K 31/7004, A61P 35/00

(54) **PROTECTION DE LA NEURITOXICITE DE L'OXALIPLATINE PAR ADMINISTRATION DE CALCIUM ET DE MAGNESIUM**
SCHUTZ GEGEN DIE NEUROTOXIZITÄT DES OXALIPLATINS DURCH VERABREICHUNG VON KALZIUM UND MAGNESIUM
PROTECTION AGAINST THE NEUROTOXICITY OF OXALIPLATIN THROUGH THE ADMINISTRATION OF CALCIUM AND MAGNESIUM

(30) Priorité: 14.01.2002 FR 0200390
(43) Date de publication de la demande: 13.10.2004
(73) Titulaire: Centre Regional de Lutte Contre le Cancer D'Angers, 49000 Angers (FR)
(72) Inventeur: GAMELIN, Laurence, F-49080 Bouchemaine (FR); GAMELIN, Erick, F-49080 Bouchemaine (FR); BOISDRON-CELLE, Michèle, F-49170 Saint-Léger-des-bois (FR); MOREL, Alain, F-49610 Juigne sur Loire (FR)
(74) Mandataire: Ahner, Francis
(86) Numéro de dépôt international: PCT/FR2003/000098
(87) Numéro de publication internationale: WO 2003/059361

(56) Documents cités:
- WO-A-01/08693
- WO-A-01/74368
- F.GROLLEAU E.A.: "A possible explanation for a neurotoxic effect of the anticancer agent oxaliplatin on neuronal voltage-gated sodium channels" JOURNAL OF NEUROPHYSIOLOGY, vol. 85, no. 5, 2001, pages 2293-2297, XP008008000
- [en ligne] Extrait de l'Internet: <URL:www.pharmacol-fr.org/nancy/info> [extrait le 2007-04-04]

## Description

La présente invention a pour objet une composition pharmaceutique à base de calcium et de magnésium qui diminue la neurotoxicité du principe actif libérant de l'oxalate lors de son métabolisme dans l'organisme, soit l'oxaliplatine.

Les dérivés du platine ont révolutionné le traitement de certains types de cancers.

Un nouveau dérivé organique du platine mis au point assez récemment, le 1,2-diamminecyclohexane (*trans-1*) oxalatoplatinum, ou oxaliplatine, s'est révélé très actif dans les tumeurs digestives (Misset et al., La Lettre du Cancérologue (1996), 5, 20-22). Il a obtenu l'AMM en première ligne thérapeutique des cancers colorectaux métastatiques. Il est à l'étude en situation adjuvante et dans d'autres tumeurs : pancréas, estomac, poumons, ovaires. Il est donc très souvent utilisé.

Dépourvu de toxicité rénale, il est à l'origine d'une neurotoxicité périphérique, qui est sa toxicité limitante. Il s'agit d'une neuropathie périphérique, très différente de celle du cisplatine, cette dernière étant chronique, cumulative, d'installation progressive et le plus souvent irréversible (Mollman, Cisplatin Neurotoxicity, (1990), 322, 126-127). La neurotoxicité de l'oxaliplatine se manifeste sous forme aiguë et/ou chronique, parfois très invalidante. L'atteinte aiguë est tout à fait originale et se caractérise par sa survenue brutale, en cours de perfusion intraveineuse ou au décours immédiat. Elle se traduit par des paresthésies des extrémités et/ou périorales, des dysesthésies exacerbées par l'exposition au froid, des contractures musculaires parfois difficilement réductibles. Les paresthésies au froid, aiguës, précoces, transitoires, siègent au niveau des doigts, des mains, de la langue et des lèvres. Elles peuvent donner un tableau de pseudoparaparésie des membres inférieurs. La neuropathie périphérique est signalée au moins une fois par 85 à 95% des patients pendant leur traitement. Elle apparaît habituellement en cours de perfusion et peut durer plusieurs jours. Sa durée augmente avec le nombre des cycles de traitement. Elles est dose dépendante et cumulative puisque la fréquence de la neuropathie de grade 3 est de 15 à 20% après une dose cumulée de 750-850 mg/m² (Berthault-Cvitkovic et al., J. Clin. Oncol. (1996), 14, 2950-8). Cette toxicité peut aller jusqu'à un pseudolaryngospasme. Cette atteinte, parfois très invalidante et angoissante, est transitoire et régresse en quelques heures ou jours.

La neuropathie d'expression chronique quant à elle, ressemble, dans ses manifestations et son évolution à celle due au cisplatine mais elle se constitue plus tardivement, après plusieurs administrations d'oxaliplatine (Machover et al., Ann. Oncol. (1996), 7, 95-8). Elle fait suite à la toxicité aiguë, peut durer plusieurs mois, voire ne pas régresser, retentissant alors sur la qualité de vie des patients qui éprouvent de graves difficultés à écrire, se boutonner, lacer leurs souliers, porter des chaussures de ville, marcher. Les études histologiques n'ont pas montré d'atteinte neuronale aussi évidente qu'avec le cisplatine, la myéline est rarement touchée, la dégénérescence Wallérienne exceptionnelle (Raymond), alors qu'avec le cisplatine, des biopsies ont montré une infiltration du tissu nerveux par le platine, avec disparition segmentaire de la gaine de myéline et dégénérescence Wallérienne (Mollman, Cisplatin Neurotoxicity, N. Engl. J. Med. (1990), 322, 126-127).

Ces problèmes de toxicité limitent l'utilisation de l'oxaliplatine et peuvent même conduire à l'arrêt du traitement. Aussi, est-il indispensable d'améliorer la tolérance de l'oxaliplatine qui s'est révélé comme indispensable dans le traitement de certains cancers.

La première étape du métabolisme de l'oxaliplatine consiste en la libération d'oxalate qui est remplacé par 2 ions chlore aboutissant ainsi à la formation du dichlorodachplatine (dichloro-diaminocyclohexane platinum). Cette réaction se fait à 30% dans le milieu plasmatique et à 70% en intracellulaire. L'oxalate est connu en toxicologie pour sa capacité de chélation du calcium et du magnésium (Hagler et al., Oxalate Metabolism, (1973), 26, 1073-1079 et L'Epée et al., Intoxication aiguë mortelle par l'oxalate de potassium. Med. Led. Dom. Corp. (1971), 4, 178-181) et une intoxication aiguë par de l'oxalate se traduit par des paresthésies, des myoclonies, pouvant aller jusqu'à des convulsions en cas d'intoxication massive. Une hypocalcémie, une acidose métabolique sont rapportées et une toxicité rénale est possible, due à la précipitation tubulaire de cristaux d'oxalate.

Aussi, les inventeurs ont-ils émis l'hypothèse du rôle de l'inhibition chronique des canaux sodiques dans la genèse de la neurotoxicité de l'oxaliplatine. En effet, les canaux ioniques jouent un rôle majeur dans l'homéostasie cellulaire neuronale et l'inhibition des canaux sodiques à long terme peut gêner les mouvements ioniques et modifier les concentrations en ions et constituants intracellulaires. Or, ceux ci sont indispensables à des processus vitaux tels que la libération de neurotransmetteurs, l'élongation du cône de croissance de l'axone, et l'expression génique. L'inhibition prolongée de la fonction neurosécrétoire et du développement neuritique peut avoir des conséquences délétères à long terme (Rizzo *et al, Mechanisms of paraesthesiae, dysesthesiae and hyperesthesiae: Role of Na Channel heterogeneity).*

Une étude électrophysiologique a été réalisée *in vitro* par:
a) des techniques de courant et potentiel imposé (voltage clamp) sur axone isolé de blatte avec la technique de double séparation d'huile,
b) des techniques de patch clamp en configuration cellule entière sur les DUM neurones (dorsal unpair median neuron) de blatte (DEA Laurence Gamelin, Etude de la neurotoxicité d'un dérivé du platine, l'oxaliplatine, par une approche électrophysiologique, Paris VII (1999)).

Sur l'axone isolé de blatte, l'oxaliplatine, appliqué par voie externe, n'a pas entraîné de modification significative des courants ioniques sodiques ni potassiques, à la différence de la tétrodotoxine, substance de référence pour étudier l'inhibition de l'ensemble des canaux sodiques. En technique de patch-clamp en configuration cellule entière, une réduction d'amplitude du potentiel d'action au cours du temps a été obtenue en présence d'oxaliplatine, en courant imposé, ainsi qu'une réduction de 50% du courant sodique en 10 minutes en potentiel imposé, atteignant un plateau de 60% de réduction du courant sodique. Les inventeurs ont pu ainsi montrer que l'oxaliplatine réduit de façon importante le courant entrant de Na, et donc réduit le potentiel d'action du neurone. Les concentrations nécessaires à l'inhibition des canaux sodiques sont de l'ordre de grandeur des concentrations obtenues dans les premières heures de perfusion de l'oxaliplatine en clinique humaine.

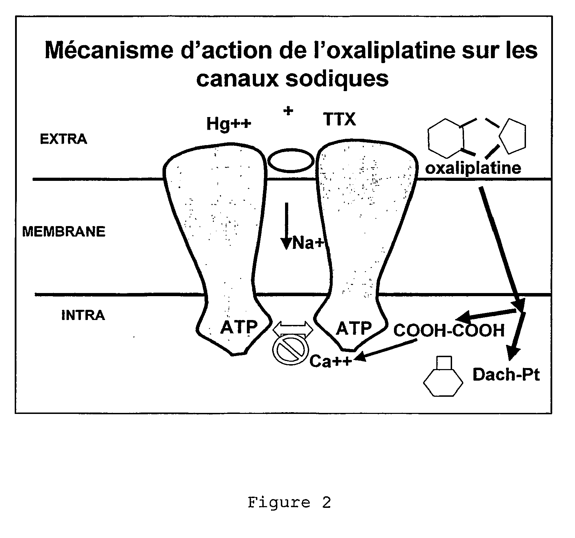

L'effet de l'oxaliplatine sur les canaux calciques a également été recherché, mais aucun effet significatif n'a été retrouvé. Il exerce une action inhibitrice certaine sur les canaux sodiques par voie intracellulaire mais son action diffère de la tétrodotoxine par une CI₅₀ moins efficace (avec un rapport de concentration de 10⁻⁴) puisque l'on obtient un plateau survenant à 60% d'inhibition. Les résultats montrent qu'il agit sur certains canaux ioniques calcium dépendants. En effet, le BAPTA, chélateur du calcium, a donné une courbe d'inhibition des canaux sodiques identique. Les inventeurs ont pu montrer que l'oxaliplatine agit par l'intermédiaire de l'oxalate, bloquant certains canaux sodiques, calcium dépendants, impliqués dans la transmission de l'influx nerveux (Figure 2). Ce blocage des canaux sodiques entraîne des anomalies du potentiel d'action du neurone, c'est à dire des troubles de la dépolarisation neuronale et potentiellement des perturbations de la transmission de l'influx.

D'autres sels de platine ont été testés: le cisplatine, le carboplatine et le Dach platine, métabolite de l'oxaliplatine n'exercent aucun effet.

Les inventeurs se sont donc donnés pour but d'utiliser du calcium et du magnésium pour augmenter la tolérance à l'oxaliplatine.

Ainsi, la présente invention a pour objet des produits comprenant du calcium, du magnésium injectable et un principe actif qui libère de l'oxalate lors de son métabolisme dans l'organisme soit l'oxaliplatine, en tant que combinaison utile pour une administration simultanée, séquentielle ou séparée en thérapeutique anticancéreuse ou antivirale.

Dans le mode de réalisation avantageux de l'invention, une partie du calcium est sous forme injectable et l'autre partie sous forme orale.

Dans un autre mode de réalisation avantageux de l'invention, le principe actif qui libère de l'oxalate est l'oxaliplatine.

Au sens de la présente invention, on entend par calcium et magnésium les formes salifiées de ces ions, notamment le gluconate de calcium, le chlorure de calcium, le bromo-galactogluconate, le gluconolactate de calcium, le carbonate de calcium, le sulfate de magnésium et le pidolate de magnésium.

Dans un mode de réalisation avantageux de l'invention, les produits sont caractérisés en ce que les concentrations en calcium injectable sont comprises entre 8 et 20 mg/ml et les concentrations en magnésium injectable sont comprises entre 10 et 20 mg/ml, de préférence 15 mg/ml (ces concentrations sont exprimées en ions calcium).

Les concentrations en sels de calcium et de magnésium sont choisies de manière à permettre une administration intraveineuse de 2 à 3 g/jour desdits sels lors de l'administration de l'oxaliplatine; les concentrations en calcium sont choisies de manière à permettre une administration de 1 à 2 g/jour *per os* les huit jours qui suivent.

L'invention a également pour objet l'utilisation du calcium et du magnésium pour la préparation d'un produit de combinaison destiné à prévenir ou à traiter la neurotoxicité causée par l'administration d'un produit qui libère de l'oxalate lors de son métabolisme, soit l'oxaliplatine

Par forme injectable, on entend au sens de la présente invention toute forme liquide capable de transporter la composition dans le corps humain d'un patient, comme par exemple les solutions isotoniques.

Par forme orale, on entend toute forme adaptée à l'administration orale, notamment les comprimés, les capsules et les solutions.

Le calcium et le magnésium peuvent être utilisés à toute concentration efficace permettant d'augmenter la tolérance de l'oxaliplatine; une perfusion dans du sérum glucosé à 5% contenant 1 g de gluconate de calcium et 1 g de sulfate de magnésium avant et après l'oxaliplatine, donne de bons résultats.

L'administration de calcium par voie orale peut se faire également à toute dose permettant d'obtenir l'effet souhaité, notamment à une dose de 1 g/jour par voie orale pendant les 8 jours qui suivent le traitement à l'oxaliplatine.

Chez des patients traités par de l'oxaliplatine pour des tumeurs digestives, 1 gramme de gluconate de calcium et 1 gramme de sulfate de magnésium ont été administrés par perfusion rapide dans du sérum glucosé à 5%, avant et après l'oxaliplatine. On a pu observer une réelle efficacité de ces perfusions d'abord en curatif des manifestations neurotoxiques aiguës, puis en préventif. La perfusion de calcium et de magnésium a fait régresser la neuropathie aiguë très rapidement et durablement. Administrée préventivement, avant et après perfusion d'oxaliplatine, elle a réduit de façon majeure la survenue de manifestations de neurotoxicité.

Chez des patients traités par oxaliplatine, on a pu montrer des concentrations significatives d'oxalate dans le plasma, normalement non détectable, ainsi qu'une élimination urinaire importante d'oxalate dans les 5 heures qui suivent le début de la perfusion d'oxaliplatine, accompagnée d'une élévation de la calciurie, de la kaliurie et de la magnésurie, mais ceci sans aucune variation des concentrations plasmatiques de calcium (total, ionisé) et de magnésium (total et globulaire). On peut donc supposer un déséquilibre fin intracellulaire dans l'homéostasie du calcium.

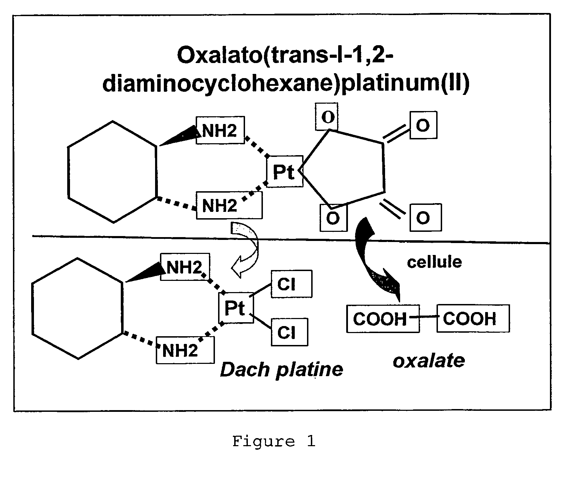

Les figures 1 à 3 qui suivent et les exemples de cas cliniques illustrent l'invention.
La figure 1 illustre le métabolisme de l'oxaliplatine. L'oxaliplatine pénètre à 70% inchangé dans la cellule et est métabolisé en diamine cyclohexane platine (Dach platine) et oxalate.
La figure 2 illustre le mécanisme d'action de l'oxaliplatine sur les canaux sodiques. L'oxaliplatine, pénètre dans la cellule et est métabolisé en donnant le Dach platine, métabolite actif cytotoxique et l'oxalate.
La figure 3 illustre la durée du traitement en fonction de la prévention par calcium et magnésium et la fréquence et l'intensité de la neuropathie distale en fin de traitement en fonction de la perfusion de calcium et de magnésium. Abréviations: Tox: effets toxiques; PD: maladie progressive; SD: maladie stable; OR: réponse objective; neuro: neuropathie; thrombo: thrombopénie; et neutro: neutropénie.

### EXEMPLES

Prévention de la neurotoxicité de l'oxaliplatine par du calcium et du magnésium.

### 1. Méthodologie.

103 traitements comportant de l'oxaliplatine ont été réalisés selon le schéma qui suit:
- FOLFOX 4: oxaliplatine 85 mg/m²/15j + fluorouracile et acide folinique (FUFOL),
- FOLFOX 6: oxaliplatine 100 mg/m²/15j + FUFOL,
- FUFOL- LOHP: oxaliplatine 130 mg/m²/21j + FUFOL, chaque traitement représentant respectivement 20%, 22% et 58% des patients traités.

On a évalué la tolérance du traitement et quantifié les manifestations toxiques en fonction de l'échelle de neurotoxicité NCI-CTC et de l'échelle spécifique de l'oxaliplatine illustrées dans le tableau 1 ci-dessous.

**Tableau 1**

| | Echelle de neurotoxicité | |
|---|---|---|
| | **NCI-CTC** | **Echelle spécifique** |
| Grade 1 | Paresthésies légères, perte des R.O.T. | Paresthésies, dysesthésies de courte durée |
| Grade 2 | Paresthésies modérées, perte sensitive objective | Paresthésies, dysesthésies persistant entre les cycles |
| Grade 3 | Paresthésies avec altération fonctionnelle, perte sensitive sévère | Paresthésies, dysesthésies avec altération fonctionnelle |

### 2. Résultats.

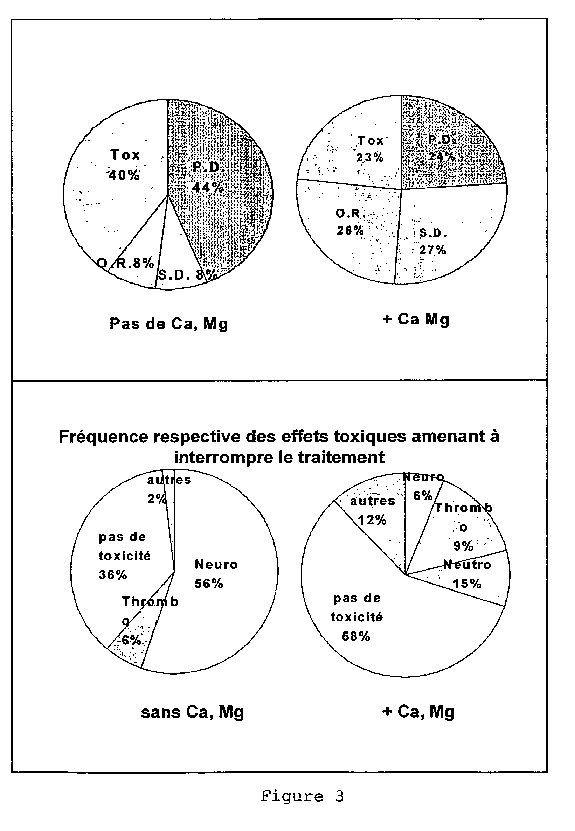

Ils sont illustrés dans la figure 3 en annexe.

Dans le groupe de patients sans calcium ni magnésium, 40% des arrêts sont dus à des causes toxiques et 44% sont liés à une progression de la maladie. Dans le groupe avec calcium et magnésium, il y a moins de toxicité et les raisons d'arrêt thérapeutique sont significativement plus faibles. On observe plus souvent une réponse ou une stabilité de la maladie. Les causes toxiques d'arrêt sont dans le groupe sans calcium et magnésium, essentiellement la neuropathie (56%), alors que dans le groupe avec calcium et magnésium, les autres toxicités classiques de la chimiothérapie apparaissent, neutropénie (15%), thrombopénie (9%). L'administration de calcium et magnésium permet d'éviter des effets toxiques et d'effectuer le traitement à pleines doses en respectant les intercures. Il modifie le profil de tolérance de l'oxaliplatine qui devient celui d'une molécule de chimiothérapie bien tolérée. Ceci entraîne une meilleure efficacité.

La perfusion de calcium et magnésium permet d'augmenter la durée totale des traitements, lorsque ceux-ci sont efficaces. A la fin de la chimiothérapie, quelle que soit sa durée, la fréquence et l'intensité de la neuropathie distale sont significativement plus basses en cas de perfusion de calcium et magnésium (p < 0,001).

Au cours du traitement, aux cycles 1, 3, 6 et à la fin du traitement, quel que soit le cycle, la fréquence et l'intensité de la neuropathie distale sont significativement inférieures dans le groupe de patients ayant reçu du calcium et du magnésium. Au cycle 6, 40% des patients n'ont pas de manifestation neurologique vis 12% en l'absence de calcium et magnésium. De même, avec du calcium et du magnésium, il n'y a pas de toxicité de grade 3, donc chronique invalidante. Le nombre de patients sans calcium ni magnésium diminue progressivement du cycle 1 au cycle 6: parce qu'un certain nombre d'entre eux reçoivent ensuite du calcium et du magnésium, parce que les autres interrompent leur traitement en raison d'une neuropathie.

Avec l'échelle spécifique, les résultats sont équivalents à l'échelle du NCI-CTC. L'échelle spécifique de l'oxaliplatine se base non seulement sur le retentissement fonctionnel de la neuropathie mais aussi sur sa durée. La perfusion de calcium et de magnésium réduit de façon significative (p < 0,001) l'intensité de la neuropathie distale, sa durée et son retentissement fonctionnel.

Cette étude a montré que:
- la perfusion de calcium et de magnésium a permis de réduire significativement l'intensité et la durée de l'ensemble des manifestations neurotoxiques aiguës de l'oxaliplatine,
- la perfusion de calcium et de magnésium a réduit aussi de façon significative la toxicité chronique, en terme d'incidence et d'intensité,
- l'oxaliplatine peut être réintroduit après traitement prolongé à une dose totale allant jusqu'à 780 mg/m², même en cas de neuropathie persistante chronique, du moment qu'il est associé à une perfusion de calcium et de magnésium.

Dans certains cas, il peut s'avérer nécessaire de poursuivre l'administration de Ca pour réduire le risque de survenue de manifestations neurologiques à distance de l'administration de l'oxaliplatine. Dans ces cas, le calcium par voie orale est efficace et permet de respecter la qualité de vie des patients.

### Cas clinique 1.

Une patiente de 58 ans est traitée par l'association 5-fluororuracile acide folinique, oxaliplatine, 130 mg/m²/21 j.

Les 2 premiers cycles, elle a une perfusion de calcium et de magnésium avant et après l'oxaliplatine. Les effets neurologiques se résument au 2ème cycle à une neuropathie périphérique de grade 1 de l'échelle spécifique.

Au 3ème cycle, la prévention par calcium et magnésium est oubliée. La patiente présente alors brutalement à la fin de la perfusion une neurotoxicité aiguë périphérique et périorale de grade 3 (NCI-CTC et échelle spécifique), des manifestations pharyngola-ryngées, des contractures musculaires notamment des mâchoires et des mains.

Le cycle suivant, la prévention par calcium et magnésium est réalisée et le traitement est très bien toléré.

De nouveau, celle-ci est oubliée au cycle 5 et les manifestations neurologiques aiguës réapparaissent, très importantes.

Le 6ème cycle sous couvert de calcium et magnésium est très bien toléré.

### Cas clinique 2.

Une autre patiente âgée de 60 ans est traitée pour un cancer du colon métastatique.

Son traitement consiste en l'association 5-FU acide folinique + oxaliplatine 130 mg/m²/21 jours.

Lors du cycle 1, apparition très rapide de paresthésies de grade 3 distales et périorales, de contractures musculaires des mâchoires et des jambes, de contracture des mains, accompagnées d'un engourdissement, d'un pseudolaryngospasme, d'une dyspnée, d'une asthénie et d'une diarrhée grade 3.

Au cours du cycle 2, les effets indésirables sont identiques, disparaissant en quelques minutes avec une perfusion de 1 g de calcium et magnésium i.v.: les paresthésies distales deviennent de grade 1 (NCI et échelle spécifique).

Au troisième cycle, sous couvert de calcium et magnésium en perfusion à visée préventive, il n'y a pas de neurotoxicité, pas de diarrhée.

### Cas clinique 3.

Un autre patient âgé de 66 ans est traité pour un cancer du colon métastatique.

Il reçoit en première ligne de chimiothérapie, la combinaison 5-FU, acide folinique + oxaliplatine à la dose de 130 mg/m²/3 semaines, sans perfusion de calcium et magnésium.

Au cours des cycles 1 et 2, apparition de paresthésies distales, périorales, de grade 2 NCI et de grade 1 de l'échelle spécifique.

Aux cycles C3, C4, C5, la neuropathie s'aggrave et devient de grade 2 NCI et 2 de l'échelle spécifique.

Au stade C6, la neuropathie continue à s'aggraver et devient de grade 3 NCI et 3 de l'échelle spécifique: neuropathie chronique, contractures des mains; retentissement clinique courant et très gênant: pour écrire, se boutonner, lacer ses chaussures. La neuropathie a duré 18 mois, très gênante et invalidante.

Deux ans plus tard, le patient constate une amélioration tout à fait significative, un de ses troubles neurologiques, la tumeur métastatique évolue de nouveau.

Le même traitement de chimiothérapie est repris, avec l'oxaliplatine selon le même schéma à 130 mg/m²/3 semaines.

Six cycles de ce protocole sont effectués, sous couvert de perfusions de calcium et magnésium avant et après perfusion d'oxaliplatine. Aucune neuropathie n'est signalée ni constatée.

## Revendications

1. Produits comprenant du calcium, du magnésium injectable et de l'oxaliplatine, en tant que combinaison utile pour une administration simultanée, séquentielle ou séparée pour une utilisation en thérapeutique anticancéreuse et antivirale, **caractérisés en ce qu'**une partie du calcium est sous forme injectable et l'autre partie sous forme orale.

2. Produits selon la revendication 1, **caractérisés en ce que** le calcium est sous forme de gluconate, de chlorure, de bromo-galactogluconate, de gluconolactate ou de carbonate et le magnésium sous forme de sulfate ou de pidolate.

3. Produits selon la revendication 2, **caractérisés en ce que** les concentrations en calcium injectable sont comprises entre 8 et 20 mg/ml (ces concentrations sont exprimées en ions calcium) et les concentrations en magnésium injectable sont comprises entre 10 et 20 mg/ml, de préférence 15 mg/ml.

4. Produits selon la revendication 2 ou 3, **caractérisés en ce qu'**ils comprennent du sérum glucosé à 5% contenant 1 g de gluconate de calcium et 1 g de sulfate de magnésium.

5. Produits selon l'une quelconque des revendications 1 à 4, **caractérisés en ce que** le calcium par voie orale est sous une forme permettant son administration à une dose de 1 g/jour par voie orale pendant les 8 jours qui suivent le traitement à l'oxaliplatine.

6. Utilisation du calcium et du magnésium pour la préparation d'un produit de combinaison destiné à prévenir ou à traiter la neurotoxicité causée par l'administration d'oxaliplatine, **caractérisée en ce qu'**une partie du calcium est sous forme injectable et l'autre partie sous forme orale.

7. Utilisation selon la revendication 6, **caractérisée en ce que** le calcium est sous forme de gluconate, de chlorure, de bromo-galactogluconate, de gluconolactate ou de carbonate et le magnésium sous forme de sulfate ou de pidolate.

8. Utilisation selon la revendication 7, **caractérisée en ce que** les concentrations en calcium injectable sont comprises entre 8 et 20 mg/ml (ces concentrations sont exprimées en ions calcium) et les concentrations en magnésium injectable sont comprises entre 10 et 20 mg/ml, de préférence 15 mg/ml.

9. Utilisation selon la revendication 7 ou 8, **caractérisée en ce que** le produit de combinaison comprend du sérum glucosé à 5% contenant 1 g de gluconate de calcium et 1 g de sulfate de magnésium.

10. Utilisation selon l'une quelconque des revendications 7 à 9, **caractérisée en ce que** le calcium par voie orale est sous une forme permettant son administration à une dose de 1 g/jour par voie orale pendant les 8 jours qui suivent le traitement à l'oxaliplatine.

## Claims

1. Products comprising calcium, injectable magnesium and oxaliplatin, as a combination useful for simultaneous, sequential or separate administration for use in anticancer or antiviral therapy, **characterized in that** part of the calcium is in injectable form and the other part is in oral form.

2. Products according to Claim 1, **characterized in that** the calcium is in gluconate, chloride, bromogalactogluconate, gluconolactate or carbonate form and the magnesium is in sulphate or pidolate form.

3. Products according to Claim 2, **characterized in that** the concentrations of injectable calcium are between 8 and 20 mg/ml (these concentrations are expressed as calcium ions) and the concentrations of injectable magnesium are between 10 and 20 mg/ml, preferably 15 mg/ml.

4. Products according to Claim 2 or 3, **characterized in that** they comprise 5% glucose serum containing 1 g of calcium gluconate and 1 g of magnesium sulphate.

5. Products according to any one of Claims 1 to 4, **characterized in that** the oral calcium is in a form allowing its administration at a dose of 1 g/day orally for the 8 days following the treatment with oxaliplatin.

6. Use of calcium and magnesium for the preparation of a combination product intended to prevent or treat the neurotoxicity caused by the administration of oxaliplatin, **characterized in that** part of the calcium is in injectable form and the other part is in oral form.

7. Use according to Claim 6, **characterized in that** the calcium is in gluconate, chloride, bromogalactogluconate, gluconolactate or carbonate form and the magnesium is in sulphate or pidolate form.

8. Use according to Claim 7, **characterized in that** the concentrations of injectable calcium are between 8 and 20 mg/ml (these concentrations are expressed as calcium ions) and the concentrations of injectable magnesium are between 10 and 20 mg/ml, preferably 15 mg/ml.

9. Use according to Claim 7 or 8, **characterized in that** the combination product comprises 5% glucose serum containing 1 g of calcium gluconate and 1 g of magnesium sulphate.

10. Use according to any one of Claims 7 to 9, **characterized in that** the oral calcium is in a form allowing its administration at a dose of 1 g/day orally for the 8 days following the treatment with oxaliplatin.

## Patentansprüche

1. Erzeugnisse umfassend Calcium, injizierbares Magnesium und Oxaliplatin als Kombination, die für eine gleichzeitige, sequentielle oder getrennte Verabreichung verwendbar ist, für eine Verwendung in der Krebstherapie und antiviralen Therapie, **dadurch gekennzeichnet, dass** ein Teil des Calciums in injizierbarer Form und der andere Teil in oraler Form vorliegt.

2. Erzeugnisse nach Anspruch 1, **dadurch gekennzeichnet, dass** das Calcium in Form von Gluconat, Chlorid, Bromo-Galactogluconat, Gluconolactat oder Carbonat und das Magnesium in Form von Sulfat oder Pidolat vorliegt.

3. Erzeugnisse nach Anspruch 2, **dadurch gekennzeichnet, dass** die Konzentrationen an injizierbarem Calcium zwischen 8 und 20 mg/ml (diese Konzentrationen sind in Calcium-Ionen ausgedrückt) und die Konzentrationen an injizierbarem Magnesium zwischen 10 und 20 mg/ml, vorzugweise 15 mg/ml betragen.

4. Erzeugnisse nach Anspruch 2 oder 3, **dadurch gekennzeichnet, dass** sie Glucoseserum 5% umfassen, das 1 g Calciumgluconat und 1 g Magnesiumsulfat enthält.

5. Erzeugnisse nach einem der Ansprüche 1 bis 4, **dadurch gekennzeichnet, dass** das oral verabreichte Calcium in einer Form vorliegt, die seine Verabreichung in einer Dosis von 1 g/Tag in oraler Verabreichung während der 8 auf die Behandlung mit Oxaliplatin folgenden Tage ermöglicht.

6. Verwendung von Calcium und Magnesium für die Herstellung eines Kombinationserzeugnisses, das zur Vorbeugung oder Behandlung der durch die Verabreichung von Oxaliplatin verursachten Neurotoxizität vorgesehen ist, **dadurch gekennzeichnet, dass** ein Teil des Calciums in injizierbarer Form und der andere Teil in oraler Form vorliegt.

7. Verwendung nach Anspruch 6, **dadurch gekennzeichnet, dass** das Calcium in Form von Gluconat, Chlorid, Bromo-Galactogluconat, Gluconolactat oder Carbonat und das Magnesium in Form von Sulfat oder Pidolat vorliegt.

8. Verwendung nach Anspruch 7, **dadurch gekennzeichnet, dass** die Konzentrationen an injizierbarem Calcium zwischen 8 und 20 mg/ml (diese Konzentrationen sind in Calcium-Ionen ausgedrückt) und die Konzentrationen an injizierbarem Magnesium zwischen 10 und 20 mg/ml, vorzugweise 15 mg/ml betragen.

9. Verwendung nach Anspruch 7 oder 8, **dadurch gekennzeichnet, dass** das Kombinationserzeugnis Glucoseserum 5% umfasst, das 1 g Calciumgluconat und 1 g Magnesiumsulfat enthält.

10. Verwendung nach einem der Ansprüche 7 bis 9, **dadurch gekennzeichnet, dass** das oral verabreichte Calcium in einer Form vorliegt, die seine Verabreichung in einer Dosis von 1 g/Tag in oraler Verabreichung während der 8 auf die Behandlung mit Oxaliplatin folgenden Tage ermöglicht.
